(19) Europäisches Patentamt
European Patent Office
Office européen des brevets

(11) Veröffentlichungsnummer: **0 001 582**
**B1**

(12) # EUROPÄISCHE PATENTSCHRIFT

(21) Anmeldenummer: **78101043.4**

(22) Anmeldetag: **30.09.78**

(51) Int. Cl.³: **C 07 D 249/06**, // D 06 L 3/12

(54) **Verfahren zur Herstellung von Bis-triazolylstilbenen**

(30) Priorität: **13.10.77 DE 2746000**

(43) Veröffentlichungstag der Anmeldung:
**0.2.05.79 Patentblatt 79/09**

(45) Bekanntmachung des Hinweises auf die Patenterteilung:
**23.07.80 Patentblatt 80/15**

(84) Benannte Vertragsstaaten:
**CH DE FR GB**

(56) Entgegenhaltungen:
**DE - A - 1 670 914**
**DE - A - 2 210 261**
**DE - A - 2 242 784**
**DE - A - 2 254 300**
**DE - A - 2 338 881**
**DE - A - 2 423 091**

(73) Patentinhaber:
**Bayer AG**
**Zentralbereich Patente, Marken und Lizenzen**
**D - 5090 Leverkusen 1, Bayerwerk (DE)**

(72) Erfinder: **Dorlars, Alfons, Dr.**
**Mozartstrasse 32**
**D - 5090 Leverkusen 1 (DE)**
**Neuner, Otto, Dr.**
**Heiligenstock 77**
**D - 5060 Bergisch Gladbach 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 582 B1

Verfahren zur Herstellung von Bis-triazolylstilbenen

Gegenstand der Erfindung ist ein Verfahren zur Herstellung von Bis-triazolylstilbenen, die in Form der freien Säure der Formel

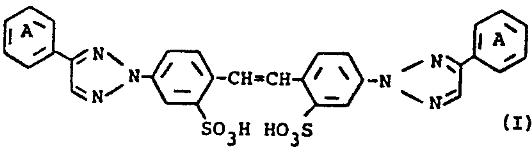

(I)

entsprechen,
worin die Phenylreste A durch Halogen, $C_1$—$C_4$-Alkyl oder $C_1$—$C_4$-Alkoxy substituiert sein können.

Geeignetes Halogen ist Brom und insbesondere Chlor.

Vorzugsweise wendet man das Verfahren zur Herstellung der Verbindung an, bei der die Phenylreste A nicht weitersubstituiert sind.

Die Verbindungen der Formel (I) sind aus der deutschen Patentschrift 1 279 636 (US—PS 3 485 831) als optische Aufheller bekannt. Sie werden durch Kondensation von 4,4'-Dihydrazinostilben-2,2'-disulfonsäure mit $\alpha$-Oximinomethylketonen, Isolierung und Trocknung der gebildeten Bis-(oximinohydrazono)-stilbendisulfonsäuren und nachfolgende Cyclisierung unter Wasserabspaltung hergestellt. In der o.g. deutschen Patentschrift wird die Cyclisierung mit einem 30-bis 40-fachen molaren Überschuß (bezogen auf Bis-(oximinohydrazono)-stilbendisulfonsäure) an Acetanhydrid als Suspensionsmedium in Gegenwart von Natriumacetat und geringen Mengen Dimethylformamid ausgeführt, wobei man etwa 8 Stunden auf 105°C erhitzt. Die Ausbeuten des Verfahrens liegen bei 30 bis 40 % der Theorie.

Es hat nicht an Versuchen gefehlt, Bis-triazolyl-stilbendisulfonsäuren der Formel (I) mit höherer Ausbeute und befriedigender Reinheit aus den genannten Bis-oximinohydrazonen herzustellen. Die im folgenden beschriebenen Verfahren brachten jedoch bei der Übertragung in den großtechnischen Maßstab stets Schwierigkeiten mit sich, die die wirtschaftliche Nutzung der Verbindungen der Formel (I) erheblich beeinträchtigten.

Nach dem Verfahren der DT—AS 1 670 914 (US—PS 3 666 758) werden die Oximinohydrazone in geschmolzenem Harnstoff 1,5 bis 2 Stunden auf 130 bis 165°C erhitzt. Die bei Mono-triazolylverbindungen durchweg zufriedenstellenden Ausbeuten werden bei den Bis-triazolyl-stilbendisulfonsäuren der Formel (I) nicht erreicht. Nachteilig an diesem Verfahren ist auch, daß aus den Oximinohydrazonen erhebliche Anteile gelber, den Wirkstoff verunreinigender Nebenprodukte entstehen, deren Entfernung eine aufwendige Reinigungsoperation erfordern, und daß das Verfahren große Mengen Ammoniak aus dem geschmolzenen Harnstoff freisetzt, die durch zusätzliche Abgaswäscher aufgefangen werden müssen.

Dieses Verfahren konnte durch die Variante der DT—OS 2 242 784 dadurch verbessert werden, daß man die Umsetzung mit einer geringeren Harnstoffmenge in Wasser oder wäßrigem Methanol durchführt. Zwar läßt sich das Verfahren einfacher führen und liefert höhere Ausbeuten als die Harnstoffschmelze, jedoch lassen sich auch in diesem Falle die guten Ausbeuten im Labormaßstab nicht auf die großtechnische Producktion übertragen.

Der Ersatz von Harnstoff durch andere Acylierungsmittel, wie sie in der DT—OS 2 210 261 (US—PS 3 965 094) mit Isocyanaten und Pyrokohlensäureestern vorgeschlagen wurden, ergibt zwar teilweise bessere Ausbeuten, die damit erzielte Ersparnis wird jedoch durch die höheren Kosten bei der Herstellung dieser Acylierungsmittel bzw. der Entfernung der aus diesen Acylierungsmitteln entstehenden Nebenprodukte mehr als kompensiert.

Es bestand daher weiterhin ein Bedürfnis nach einem wirtschaftlichen Verfahren zur Herstellung der optischen Aufheller der Formel (I). Es wurde nun gefunden, daß man Bis-triazolylstilbenverbindungen der Formel (I) in hoher Reinheit, mit hoher Ausbeute und in wirtschaftlicher Weise großtechnisch dadurch herstellen kann, daß man Oximinohydrazone der Formel

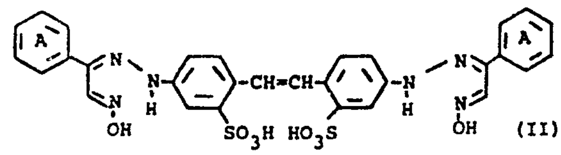

(II)

worin A wie angegeben substituiert sein kann, in Gegenwart von Harnstoff und polaren Lösungsmitteln bei Temperaturen von 10 bis 60°C, vorzugsweise 25 bis 45°C, mit Anhydriden niederer Carbonsäuren umsetzt und das Reaktionsgemisch gegenbenenfalls auf 70 bis 90°C nacherhitzt.

Die Oximinohydrazone der Formel (II) sind bekannt. Man erhält sie leicht durch Kondensation von 4,4'-Dihydrazinostilben-2,2'-disulfonsäure mit den entsprechenden Oximinomethylarylketonen. Nach dem erfindungsgemäßen Verfahren ist eine Zwischenisolierung der so hergestellten Oximinohydrazone nicht erforderlich, vielmehr kann man nach beendeter Kondensation ohne Unterbrechung des Fabrikationsablaufes die Cyclisierung vornehmen.

Als Anhydride niederer aliphatischer Carbonsäuren kommen einfache als auch gemischte Anhydride von Carbonsäuren mit 1 bis 4 C-Atomen in Frage, beispielsweise Ameisensäure-Essigsäure-Anhydrid, Propionsäureanhydrid und vorzugsweise Acetanhydrid.

Die polaren Lösungsmittel können Alkohole, Äther, Ester, Carbonsäureamide, Sulfoxide, Sulfone und Phosphorsäureamide sein, beispielsweise Dimethylformamid, Dimethylsulfoxid, Glykolmethylätheracetat, Glykoldimethyläther, Methanol, Äthanol, n- und i-Propanol, Butanol, Glykol, Glykolmonomethyläther, Glykolmonoäthyläther, Diglykol, Diglykolmonomethyl- und -äthyläther, Tri- und Tetraglykol sowie deren Monoalkyläther.

Der erfindungsgemäß einzusetzende Harnstoff wirkt unter den vorliegenden Reaktionsbedingungen im Gegensatz zu den Verfahren der DT—AS 1 670 914 und der DT—OS 2 242 784 nicht als Acylierungsmittel.

Für die Cyclisierung eines Moles an Verbindung der Formel (II) werden 2 Mol Anhydrid gebraucht; zweckmäßigerweise arbeitet man jedoch mit einem Überschuß an Anhydrid, d.h. man setzt auf 1 Mol zu cyclisierende Verbindung (II) 2 bis 4 Mol Anhydrid ein. Größere Überschüsse stören nicht, sind jedoch aus ökonomischen Gründen nicht zu empfehlen.

Bezogen auf die Menge des Oximinohydrazons, verwendet man die 1- bis 4-fache Menge an Gemisch aus Harnstoff und polarem Lösungsmittel, welches aus 10 bis 90 Gew.-% Harnstoff und 90 bis 10 Gew.-% polarem Lösungsmittel besteht. Die Reaktionsmischung kann darüber hinaus noch bis zu 50 %, bezogen auf die Menge des Oximinohydrazons, an Wasser enthalten. Höhere Wasseranteile im Reaktionsgemisch sollten vermieden werden, um die Triazolausbeuten nicht fühlbar zu vermindern. Andererseits braucht nicht unbedingt wasserfrei gearbeitet zu werden, so daß die für die Oximinohydrazonherstellung erforderlichen Vorprodukte Dihydrazinostilbendisulfonsäure und das jeweils verwendete Oximinoketon als noch feuchte, 20 bis 30 % Wasser enthaltende Preßpasten, wie sie in der Fabrikation anfallen, ohne Trocknung eingesetzt werden können. Bei stärker wasserhaltigen Ausgangsmaterialien zieht man wenigstens einem Teil des Wassers unter vermindertem Druck ab. Die Konzentration des Oximinohydrazons im Cylisierungsansatz wird man möglichst hoch wählen; sie wird lediglich begrenzt durch die Forderung nach guter Durchmischung und Rührbarkeit des Reaktionsansatzes während der Zugabe des Carbonsäureanhydrids. Der Einsatz eines geeigneten wirksamen Rührwerks ist daher von besonderer Wichtigkeit. Selbstverständlich kann man auch wesentlich verdünnter unter Erhöhung des Produkt/Lösungsmittel-Harnstoff-Verhältnisses arbeiten, ohne daß schlechtere Ausbeuten oder weniger reine Produkte erhalten werden. Allerdings ist dies aus ökonomischen Gründen nicht zu empfehlen. Hingegen kann der Zusatz wirksamer Emulgatoren, beispielsweise vom Typ Phenolpolyglykoläther, alkylierter Phenolpolyglykoläther und deren Schwefelsäurehalbester, von Nutzen sein.

Bei der praktischen Ausführung des erfindungsgemäßen Verfahrens verfährt man beispielsweise so, daß man Dihydrazinostilbendisulfonsäure als feuchte Paste in ein vorgelegtes Alkohol-Harnstoff-Gemisch einrührt und nach analytischer Gehaltsbestimmung die berechnete Menge des Oximinoketons zugibt. Sobald die Kondensation beendet ist, kühlt man unter Rühren auf 20 bis 35°C ab und stellt den pH-Wert durch Zutropfen von Alkalilauge auf einen Wert zwischen 7 und 10, vorzugsweise auf etwa 8,0 bis 8,5 (mit der Glaselektrode bestimmt). Unter Einhaltung dieses Wertes und der Temperatur läßt man einen geringen Überschuß über die dem Oximinoketon entsprechende molare Menge des Carbonsäureanhydrids unter Rühren zutropfen. Dabei cyclisiert der überwiegende Teil der Oximinogruppen zum Triazol; um auch letzte Anteile zu cyclisieren und um ferner eine günstige, leicht filtrierbare Kristallform zu erreichen, erwärmt das Gemisch nach kurzer Nachrührzeit auf 70 bis 90°C, gewünschtenfalls nach Zugabe von Wasser. Bei Einsatz eines niedrigsiedenden Alkohols wie Methanol oder Äthanol läßt sich dieser dabei, gegebenenfalls über eine Trennsäule, abdestillieren und wieder verwenden. Das als orange-gelbe Suspension vorliegende Reaktionsprodukt der Formel (I) ist dann leicht über eine Filterpresse zu isolieren. Anschließend wird es in bekannter Weise gereinigt. Es kann schließlich je nach Verwendungszweck in die freie Säure oder in ein anderes Salz übergeführt werden.

Die Verbindungen der Formel (I), sowie deren Alkalisalze stellen wertvolle Weißtöner für Textilien und Waschmittel dar.

### Beispiel 1

In einem heizbaren 1 $m^3$-VA-Kessel mit wandgängigem Ankerrührer werden in vorgelegte 82 kg Diglykolmethyläther unter Rühren 80 kg 70 %ige 4,4'-Dihydrazinostilben-2,2'-disulfonsäure (feuchte Preßpaste) eingetragen. Darauf werden 82 kg Harnstoff und 29 kg wasserfreies Kaliumacetat zugesetzt. Sobald eine homogene Suspension vorliegt, werden unter weiterum Rühren 56,5 kg 75 %iges Oximinoacetophenon (feuchte Filterware) zugegeben. Der Kesselinhalt wird auf 50°C erwärmt; allmählich stellt sich ein pH-Wert von 4,8 bis 5,0 ein. Der größte Teil des eingeschleppten Wassers destilliert nach Anlegen von Vakuum (ca. 25 mbar) ab. Diese Temperatur wird bis zur Beendigung der Kondensation (etwa 1 Stunde) gehalten; schließlich setzt man 1 kg eines Aralkyl-phenolpolyglykoläther-sulfonats, gelöst in 1,5 Liter Wasser,· hinzu kühlt die orangefarbene Suspension des Oximinohydrazons auf 28 bis 32°C und stellt den pH-Wert durch Zutropfen von 50 %iger Kalilauge auf 7,8 bis 8,2. Unter Einhaltung dieser Temperatur (Kühlung) und pH-Werte (Zutropfen von Kalilauge) läßt man allmählich 32 kg Acetanhydrid innerhalb etwa 1 1/2 Stunden zufließen und rührt noch 1/2 Stunde nach. Nach Zufluß von 500 Litern Wasser wird das Gemisch auf 80 bis 90°C erhitzt und 1 bis 2 Stunden bei dieser Temperatur nachgerührt. Darauf läßt man

auf 75°C abkühlen, drückt den Kesselinhalt durch eine Filterpresse, wäscht mit 300 Liter 3 %iger Kaliumchloridlösung und schließlich mit 140 Liter Wasser nach. Man erhält etwa 300 kg einer gelblichen Preßpaste, die man in bekannter Weise durch Umlösen aus heißer wäßriger verdünnter Kalilauge reinigt. Nach dem Trocknen erhält man so 87,5 kg des Di-kaliumsalzes der Formel (I) (Ring A unsubstituiert) als 93,5 %ige Ware.

Ausbeute: 83 % der Theorie.

Ähnlich gute Ausbeuten werden erhalten, wenn man Diglykolmethyläther durch die gleiche Menge Glykoldimethyläther, Glykolmethylätheracetat oder Dimethylformamid ersetzt.

Beispiel 2

In einem 10 Liter-Glaskolben, versehen mit einem gut wirksamen Rührer, werden in eine vorgelegte Mischung aus 1 kg Harnstoff und 1 Liter Methanol 400 g 4,4′-Dihydrazinostilben-2,2′-disulfonsäure (100 %ig) als ca. 75 %ige Paste eingerührt. Man trägt zunächst 185 g Natriumacetat und darauf 304 g Oximinoacetophenon (100 %ig) als ca. 80 %ige feuchte Ware ein. Nach etwa 2-stündigem Rühren bei 50°C und pH 4,7 bis 5,0 (gemessen mit der Glaselektrode) ist die Kondensation beendet. Man kühlt auf etwa 30°C ab und stellt bei dieser Temperatur den pH-Wert mit 50 %iger Kalilauge auf 7,6 bis 8,0 ein. Unter Einhaltung dieser Bedingungen (Außenkühlung, Zutropfen von Kalilauge) tropft man innerhalb einer Stunde unter gutem Rühren 240 g Acetanhydrid hinzu. Man rührt etwa 15 Minuten nach, wobei das orangefarbene Reaktionsgemisch sehr gut rührbar bleibt. Darauf läßt man 3 l Wasser einfließen, wobei man mit Kalilauge pH 7,5 einstellt, und erwärmt das Gemisch unter weiterem Rühren allmählich bis auf 90 bis 98°C, wobei wasser- und ammoniakhaltiges Methanol abdestilliert, das nach einer Rektifikation wieder verwendet werden kann. Schließlich läßt man auf 80 bis 85°C erkalten, saugt das orange-gelbe Produkt auf einer Nutsche ab, wäscht mit 3 Liter 2 %iger Kochsalzlösung und gibt 1,5 Liter kaltes Wasser dazu. Man erhält 2,4 kg einer wäßrigen Paste, die man zur weiteren Reinigung in 8 Liter Wasser unter Zusatz von 20 ml 40 %iger Natronlauge kochend löst. Man kocht 2 Stunden unter Rückflußkühlung und gibt dann 500 g Natriumchlorid hinzu. Anschließend läßt man auf 85°C erkalten, saugt die ausgeschiedenen gelben Kristalle ab und wäscht sie mit 3 Liter 1 %iger Kochsalzlösung. Nach dem Trocknen erhält man 590 g 91 %iges Di-natriumsalz der Formel (I) (A nicht weitersubstituiert), entsprechend einer Ausbeute von 80 % der Theorie.

Ähnliche Ergebnisse werden erhalten, wenn man etwa die Hälfte der Kalilauge durch Natronlauge ersetzt. Verwendet man anstelle des Oximinoacetophenons 336 g p-Methyloximinoacetophenon, 370 g p-Chloroximinoacetophenon oder 365 g p-Methoxyoximinoacetophenon, so erhält man die betreffenden, in p-Stellung von A durch Methyl, Chlor oder Methoxy substituiertun Verbindungen der Formel (I) in vergleichbaren Ausbeuten.

## Patentansprüche

1. Verfahren zur Herstellung von Bis-triazolylstilbenverbindungen, die in Form de freien Säure der Formel

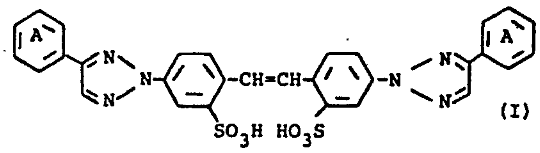

(I)

entsprechen,
worin die Phenylreste A durch Halogen, $C_1$—$C_4$-Alkyl oder $C_1$—$C_4$-Alkoxy substituiert sein können,
aus Bis-oximinohydrazonostilbenverbindungen der Formel

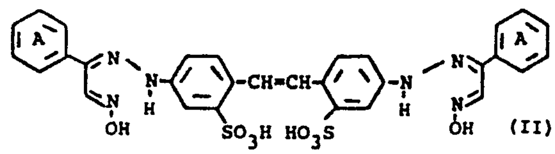

(II)

dadurch gekennzeichnet, daß man die bis-oximinohydrazonostilbenverbindungen in Gegenwart von Harnstoff und polaren Lösungsmitteln bei Temperaturen von 10 bis 60°C mit Anhydriden niederer Carbonsäuren umsetzt.

2. Verfahren nach Anspruch 1, dadurch gekennzeichnet, daß man die Umsetzung bei 25 bis 45°C durchführt.

## Claims

1. Process for the preparation of bis-triazolylstilbene compounds which, in the form of the free acid, correspond to the formula

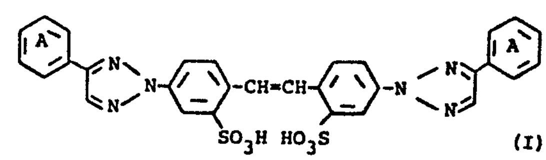

(I)

wherein
the phenyl radicals A can be substituted by halogen, $C_1$—$C_4$-alkyl or $C_1$—$C_4$-alkoxy,
from bis-hydroxyiminohydrazonostilbene compounds of the formula

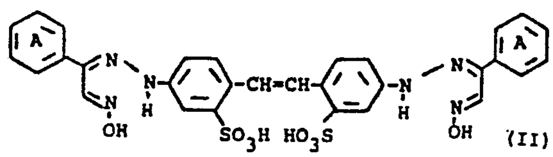

(II)

characterised in that the bis-hydroxyiminohydrazonostilbene compounds are reacted with anhydrides of lower carboxylic acids in the presence of urea and polar solvents at temperatures from 10 to 60°C.

2. Process according to Claim 1, characterised in that the reaction is carried out at 25 to 45°C.

## Revendications

1. Procédé de préparation de dérivés de bis-triazolylstilbènes qui, à l'état d'acides libres, répondent à la formule:

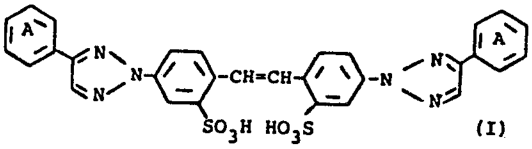

(I)

dans laquelle les noyaux phényle A peuvent être substitués par des halogènes, des groupes alkyle en $C_1$—$C_4$ ou alcoxy en $C_1$—$C_4$, à partir de dérivés de bis-oximinohydraxonostilbènes répondant à la formule:

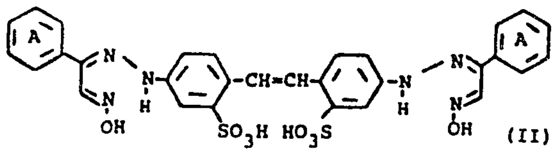

(II)

ce procédé se caractérisant en ce que l'on fait réagir les dérivés de bis-oximinohydrazono-stilbènes en présence d'urée et de solvants polaires à des températures de 10 à 60°C avec des anhydrides d'acides carboxyliques inférieurs.

2. Procédé selon la revendication 1, caractérisé en ce que l'on effectue la réaction à une température de 25 à 45°C.